# EUROPEAN PATENT SPECIFICATION

(11) **EP 2 074 080 B1**
(45) Date of publication and mention of the grant of the patent: **16.09.2015**
(21) Application number: 07816191.6
(22) Date of filing: 16.10.2007
(51) Int. Cl.: C07C 43/178, C07C 47/277, C11B 9/00

(54) **2, 3, 3 -TRIMETHYLCYCLOPENT-3 -ENECARBALDEHYDE DERIVATIVES USEFUL AS ODORANTS**
ALS GERUCHSSTOFFE GEEIGNETE 2,3,3-TRIMETHYLCYCLOPENT-3-ENCARBALDEHYDDERIVATE
DÉRIVÉS DE 2, 3, 3-TRIMÉTHYLCYCLOPENT-3-ÈNECARBALDÉHYDE UTILES COMME SUBSTANCES ODORANTES

(30) Priority: 18.10.2006 GB 0620556
(43) Date of publication of application: 01.07.2009
(73) Proprietor: Givaudan SA, 1214 Vernier (CH)
(72) Inventor: BAJGROWICZ, Jerzy A., 8053 Zürich (CH); KRAFT, Philip, 8600 Dübendorf (CH)
(74) Representative: Sievert, Claudia
(86) International application number: PCT/CH2007/000508
(87) International publication number: WO 2008/046239

(56) References cited:
- EP-A- 0 801 049
- US-A- 4 052 341

## Description

The present invention refers to a novel class of 2,2,3-trimethylcyclopent-3-enecarbaldehyde derivatives useful as odorants. This invention relates furthermore to a method for their production and to fragrance compositions comprising them.

United states patent 4,052,341 discloses 3-methyl-5-(2,2,3-trimethyl-cyclopent-3-en-1-yl)pentan-2-ol and perfume compositions. European patent application EP 0 801 049 relates to cyclopentanebutanol derivatives as odorants.

In the fragrance industry there is a constant demand for new compounds that enhance, modify or improve on odour notes. Surprisingly, a new class of compounds was found, possessing woody, sandalwood, ambery, patchouli, floral, green, lactonic notes.

Accordingly, the present invention refers in one of its aspects to compounds of formula (I) 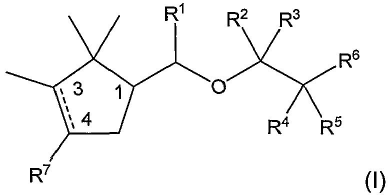 wherein:
R¹, R², R³, and R⁴ are independently selected from hydrogen, methyl or ethyl;
R⁵ is hydrogen, methyl or ethyl; and R⁶ is hydroxyl; or
R⁵ and R⁶ form together with the carbon atom to which they are attached a carbonyl group;
R⁷ is hydrogen and the bond between C-3 and C-4 is a single bond or the dotted line together with the bond between C-3 and C-4 represents a double bond; or
R⁷ is methylene forming with C-3 and C-4 a cyclopropane ring.

The compounds of formula (I) may comprise several chiral centres and as such may exist as a mixture of stereoisomers, or they may be resolved as isomerically pure forms. Resolving stereoisomers adds to the complexity of manufacture and purification of these compounds, and so it is preferred to use the compounds as mixtures of their stereoisomers simply for economic reasons. However, if it is desired to prepare individual stereoisomers, this may be achieved according to methods known in the art, e.g. preparative HPLC and GC, crystallization or by departing from chiral starting materials, e.g. starting from enantiomerically pure or enriched raw materials such as terpenoids, and/or by applying stereoselective synthesis.

Thus, the present invention refers in a further aspect to compounds of formula (I) enriched in either a compound of formula (A) or (B), preferably in the ratio from about 1:9 to about 9:1, e.g. about 3:2 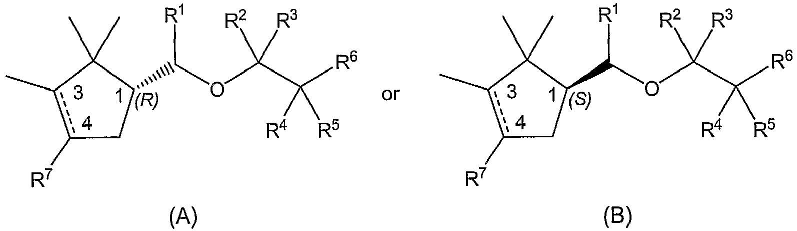 wherein R¹ to R⁷ have the same meaning as given for formula (I) above.

Particularly preferred compounds of formula (I) are
2-methyl-2-[(2,2,3-trimethylcyclopent-3-enyl)methoxy]propan-1-ol,
2-methyl-2-[(2,2,3-trimethylcyclopent-3-enyl)methoxy]propanal,
3-methyl-3-[(2,2,3-trimethylcyclopent-3-enyl)methoxy]butan-2-ol,
3-[1-(2,2,3-trimethylcyclopent-3-enyl)ethoxy]butan-2-ol,
3-[(2,2,3-trimethylcyclopent-3-enyl)methoxy]butan-2-ol,
1-[(2,2,3-trimethylcyclopent-3-enyl)methoxy]butan-2-ol,
2-[(2,2,3-trimethylcyclopent-3-enyl)methoxy]butan-1-ol,
2-methyl-2-[1-(2,2,3-trimethylcyclopent-3-enyl)ethoxy]propan-1-ol,
1-[1-(2,2,3-trimethylcyclopent-3-enyl)ethoxy]propan-2-ol ,
2-[1-(2,2,3-trimethylcyclopent-3-enyl)ethoxy]propan-1-ol,
2-methyl-2-[(1,2,2-trimethylbicyclo[3.1.0]hex-3-yl)methoxy]propan-1-ol, and
2-methyl-2-[(2,2,3-trimethylcyclopentyl)methoxy]propan-1-ol.

The compounds according to the present invention may be used alone or in combination with known odorant molecules selected from the extensive range of natural and synthetic molecules currently available, such as essential oils and extracts, alcohols, aldehydes and ketones, ethers and acetals, esters and lactones, macrocycles and heterocycles, and/or in admixture with one or more ingredients or excipients conventionally used in conjunction with odorants in fragrance compositions, for example, carrier materials, and other auxiliary agents commonly used in the art.

The following list comprises examples of known odorant molecules, which may be combined with the compounds of the present invention:
- essential oils and extracts, e.g. tree moss absolute, basil oil, fruit oils such as bergamot oil and mandarine oil, myrtle oil, palmarose oil, patchouli oil, petitgrain oil, jasmine oil, rose oil, sandalwood oil, wormwood oil, lavender oil or ylang-ylang oil;
- alcohols, e.g. cinnamic alcohol, cis-3-hexenol, citronellol, Ebanol^{®} (3-methyl-5-(2,2,3-trimethyl-3-cyclopenten-1-yl)-4-penten-2-ol), eugenol, farnesol, geraniol, Super Muguet™ (6-ethyl-3-methyl-6-octen-1-ol), linalool, menthol, nerol, phenylethyl alcohol, rhodinol, Sandalore^{®} (5-(2,2,3-trimethyl-3-cyclopentenyl)-3-methylpentan-2-ol), terpineol or Timberol^{®} (1-(2,2,6-trimethylcyclohexyl)hexan-3-ol).
- aldehydes and ketones, e.g. anisaldehyde, α-amylcinnamaldehyde, Georgywood™ (1-(1,2,8,8-tetramethyl-1,2,3,4,5,6,7,8-octahydronaphthalen-2-yl)ethanone), hydroxycitronellal, Iso E Super^{®} (1-(2,3,8,8-tetramethyl-1,2,3,4,5,6,7,8-octahydronaphthalen-2-yl)ethanone), Isoraldeine^{®} (4-(2,6,6-trimethyl-2-cyclohexenyl)-3-methyl-3-buten-2-one), Hedione^{®} (methyl (3-oxo-2-pentylcyclopentyl)acetate), Lilial^{®} (3-(4-tert-butylphenyl)-2-methylpropanal), maltol, methyl cedryl ketone, methylionone, verbenone or vanillin;
- ethers and acetals, e.g. Ambrox^{®} (3a,6,6,9a-tetramethyldodecahydronaphtho[2,1-b]furan), geranyl methyl ether, rose oxide or Spirambrene (2,2,3',7',7'-pentamethylspiro(1,3-dioxan-5,2'-norcarane)).
- esters and lactones, e.g. benzyl acetate, cedryl acetate, γ-decalactone, Helvetolide^{®} (2-[1-(3,3-dimethylcyclohexyl)ethoxy]-2-methylpropan-1-ol propanoate), γ-undecalactone or vetivenyl acetate.
- macrocycles, e.g. ambrettolide, ethylene brassylate or Exaltolide^{®} (oxacyclohexadecan-2-one).
- heterocycles, e.g. isobutylquinoline.

The compounds of the present invention may be used in a broad range of fragrance applications, e.g. in any field of fine and functional perfumery, such as perfumes, household products, laundry products, body care products and cosmetics. The compounds can be employed in widely varying amounts, depending upon the specific application and on the nature and quantity of other odorant ingredients. The proportion is typically from 0.001 to 20 weight percent of the application. In one embodiment, compounds of the present invention may be employed in a fabric softener in an amount of from 0.001 to 0.05 weight percent. In another embodiment, compounds of the present invention may be used in an alcoholic solution in amounts of from 0.1 to 30 weight percent, more preferably between 5 and 20 weight percent. However, these values are given only by way of example, since the experienced perfumer may also achieve effects or may create novel accords with lower or higher concentrations, e.g., up to about 50 weight percent based on the fragrance composition.

The compounds of the present invention may be employed into the fragrance application simply by directly mixing the fragrance composition with the fragrance application, or they may, in an earlier step, be entrapped with an entrapment material such as polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the application.

Thus, the invention additionally provides a method of manufacturing a fragrance application and consumer products resulting therefrom. The method comprises the incorporation therein of a compound of formula (I) as a fragrance ingredient, either by directly admixing the compound to the application or by admixing a fragrance composition comprising a compound of formula (I), which may then be mixed to a fragrance application, using conventional techniques and methods. Through the addition of an olfactorily acceptable amount of a compound of the present invention, the odor notes of a fragrance application will be improved, enhanced or modified.

Thus, the invention furthermore provides a method for improving, enhancing or modifying a fragrance application through the addition thereto of an olfactory acceptable amount of a compound of formula (I), or a mixture thereof.

The invention also provides a fragrance application comprising:
a) as odorant a compound of formula (I) or a mixture thereof; and
b) a consumer product base.

As used herein, "fragrance application" means any products, such as fine fragrances, e.g. eaux de perfume and eaux de toilette; household products, e.g. detergents for dishwasher, surface cleaner, air freshener; laundry products, e.g. softener, bleach, detergent; body care products, e.g. after-shave lotion, shampoo, shower gel, shower and bath salt, hygiene product; and cosmetics, e.g. deodorants, vanishing cremes, comprising an odorant. This list of products is given by way of illustration and is not to be regarded as being in any way limiting.

As used herein, "fragrance composition" means any composition comprising at least one odorant molecule and a diluent conventionally used in conjunction with odorants in fragrance compositions, such as dipropylenglycol (DPG), isopropylmyristate (IMP), triethylcitrate (TEC) and alcohol (e.g. ethanol).

The compounds of the present invention may be prepared according to Scheme 1, starting from the commercially available α-campholenic aldehyde of any enantiomer ratio (pure (R) or (*S*) or any mixture of both enantiomers, e.g. from about 9:1 to about 1:9 (R/S)) *via* the corresponding quality of its lower homologue, *α*-campholytic aldehyde (1), described in the literature by Y. Bessière-Chrétien et al., Comptes Rendus des Séances de l'Académie des Sciences, Série C: Sciences Chimiques (1971), 273(3), 272 - 275 . The latter may be reduced to the known *α*-campholytic alcohol (**2**; R¹=H), or reacted with a Grignard reagent to give a 1-substituted *α*-campholytic alcohol **2.** The abovementioned alcohols **2** reacted with a substituted oxirane in the presence of a base e.g. sodium hydride or an acid e.g. tin tetrachloride give the corresponding 2- [(2,3,3-trimethylcyclopent-3-enyl)methyloxy]ethanols **3.** The oxirane ring opening is rarely highly regioselective; mixtures of regioisomers (**3** + **3**' in which the pairs of geminal substituents R² + R³ and R⁴ + R⁵ are permuted) are usually obtained. In a case when at least one of the 1-substituents (R⁴ and/or R⁵) is hydrogen, **3** may be oxidised to the corresponding aldehyde or ketone **4** and again reacted with an organometallic reagent, e.g. Grignard reagent, to give a differently substituted **3.** The alcohols **3** may be cyclopropanated or hydrogenated to **5** and **6** respectively by the methods known in the art. The alcohols **5** and **6** may also be prepared by first cyclopropanating or hydrogenating the *α*-campholytic alcohols **2** and then carrying out the oxirane opening step, optionally followed by the oxidation and organometallic reagent addition steps. Preparations of **3, 5** and **6** may also include reacting *α*-campholytic aldehyde or its cyclopropanated or hydrogenated analogue with a substituted ethylene glycol and reducing the resulting dioxolane. Still another way to make alcohols **3, 5** and **6** consists of transforming campholytic alcohols **2** or their cyclopropanated or hydrogenated analogues into the corresponding halides and submitting them to the Williamson reaction. 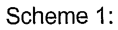 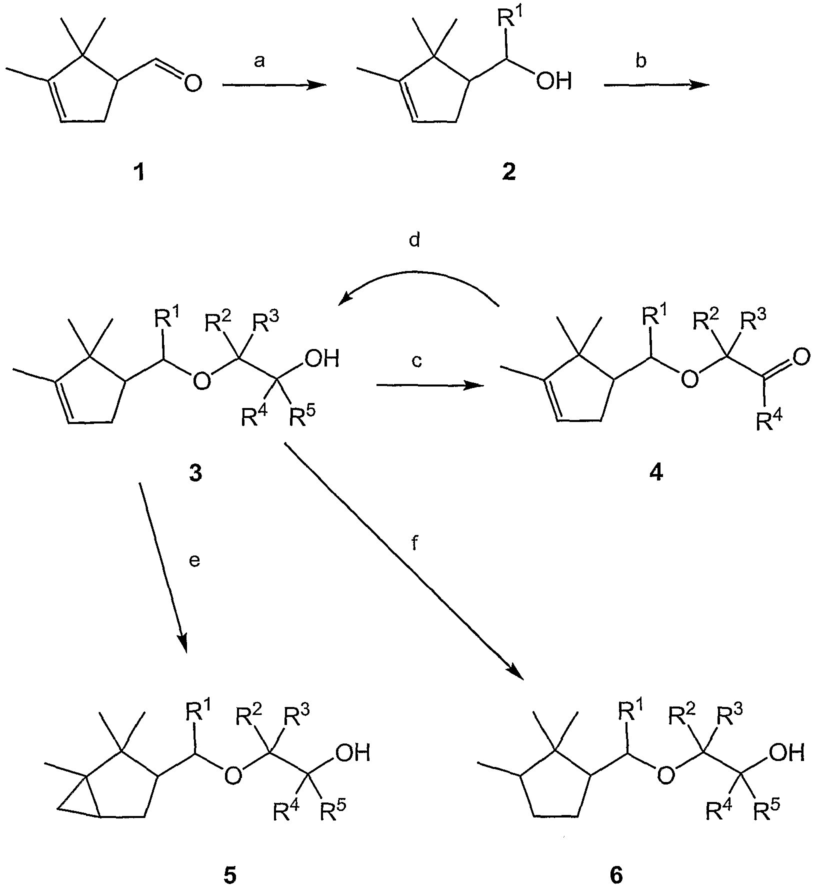 a) NaBH₄ or R¹MgX; wherein X=halogen, e.g. Cl, Br, I; b) appropriately substituted oxirane, SnCl₄ or NaH; c) Dess - Martin periodinane; d) R⁵MgX; e) CH₂I₂, Et₃Al; or CH₂Br₂, Zn, CuBr; f) H₂, Pd/C; R¹ to R⁵ have the same meaning as given for formula (I).

The invention is now further described with reference to the following non-limiting examples. These examples are for the purpose of illustration only and it is understood that variations and modifications can be made by one skilled in the art.

All products described in the Examples were obtained starting from commercially available qualities of *α*-campholenic aldehyde of 9:1 or 2:3 (R/S) enantiomer ratios.

They were transformed into the corresponding enantiomeric mixtures of known *α-*campholytic aldehyde (1). Flash chromatography: *Merck* silica gel 60 (230 - 400 mesh). The reported NMR spectra were measured in CDCl₃; chemical shifts (*δ*) are reported in ppm downfield from TMS; coupling constants *J* in Hz.

### Example 1: 2-Methyl-2-[(2,2,3-trimethylcyclopent-3-enyl)methoxy]propan-1-ol (3a); (1"R/S)=3:2

Tin tetrachloride (0.65 g, 2.5 mmol) was added to an ice-cold solution of isobutylene oxide (4.5 g, 63 mmol) in *α*-campholytic alcohol (**2a,** (*R*/*S*) = 3:2, 35.5 g, 0.25 mol) at 15°C. The temperature of the reaction mixture rose to 25°C. After further 1.5 h stirring at 15°C, the reaction mixture was poured into water, basified with saturated aqueous sodium bicarbonate solution and the aqueous layer extracted with MTBE (2 x). The combined organic phases were washed with water to pH 7, dried (MgSO₄) and concentrated. The residue (38.8 g) was distilled under reduced pressure to give the unreacted starting alcohol (24.0 g) and crude product (8.9 g) which was further purified by flash chromatography (MTBE/hexane 1:3) to afford 2-methyl-2-[(2,2,3-trimethylcyclopent-3-enyl)methoxy]propan-1-ol **(3a**, 7.2 g, 54% yield, colourless liquid).
¹H NMR: *δ*5.20 (sb, 1H), 3.49 (dd, J = 8.6, 6.8, 1 H), 3.43 (d, *J_{AB}* = 10.9, 1H), 3.39 (d, *J_{AB} =* 10.9, 1 H), 3.34 (dd, J = 8.6, 7.6, 1 H), 2.30 (m, 1 H), 2.21(sb, 1 H), 2.06 (m, 1H), 1.90 (m, 1H), 1.59 (s, 3H), 1.17 (s, 3H), 1.16 (s, 3H), 1.06 (s, 3H), 0.85 (s, 3H). ¹³C NMR: *δ* 148.2 (s), 121.3 (d), 74.6 (s), 69.9 (t), 62.6 (t), 49.7 (d), 46.4 (s), 33.7 (t), 26.9 (q), 21.9 (q), 21.6 (q), 19.8 (q), 12.2 (q). MS: 197(1), 181(4), 139(5), 123(73), 122(46), 107(100), 95(23), 91(18), 81(47), 73(18), 55(26), 43(19), 41(19).
Odour description: sandalwood, floral, fatty, suede, creamy.

### Example 2: 2-Methyl-2-[(2,2,3-trimethylcyclopent-3-enyl)methoxy]propanal (4a); (1"R/S) = 3:2

Dess - Martin periodinane (18.5 g, 44 mmol) was added portionwise to a solution of 2-methyl-2-[(2,2,3-trimethylcyclopent-3-enyl)methoxy]propan-1-ol **(3a)** from Example 1 (5.7 g, 27 mmol) in dichloromethane (75 ml) under argon. After further 30 min. stirring at room temperature, the reaction mixture was poured into ice-water, basified with saturated aqueous sodium bicarbonate solution, diluted with hexane and filtered. The separated organic layer was washed with water (2 x), dried (MgSO₄), concentrated in vacuo and purified by flash chromatography (MTBE/hexane 1:9) to afford 2-methyl-2-[(2,2,3-trimethylcyclopent-3-enyl)methoxy]propanal (**4a**, 2.0 g, 35% yield, colourless oil).
¹H NMR: *δ* 9.59 (s, 1H), 5.20 (m, 1H), 3.53 (dd, *J* = 8.6, 6.8, 1 H), 3.32 (dd, *J* = 8.6, 7.7 1 H), 2.33 (m, 1 H), 2.14 (dtd, J = 9.1, 7.8, 7.1, 1 H), 1.92 (m, 1 H), 1.59 (dt, *J* = 2.5, 1.6, 3H), 1.27 (s, 3H), 1.26 (s, 3H), 1.08 (s, 3H), 0.83 (s, 3H). ¹³C NMR: *δ* 204.8 (d), 148.1 (s), 121.2 (d), 79.6 (s), 65.3 (t), 49.7 (d), 46.4 (s), 33.6 (t), 26.7 (q), 20.9 (q), 20.4 (q), 19.7 (q), 12.2 (q). MS: 210 (M⁺, 1), 181(8), 123(100), 122(25), 107(49), 95(15), 91(16), 81(65), 79(16), 67(15), 57(18), 4(21), 41(21).
Odour description: woody, aldehydic, green, metallic, sandalwood, creamy, fatty, lactonic, watery.

### Example 3: 3-Methyl-3-[(2,2,3-trimethylcyclopent-3-enyl)methoxy]butan-2-ol; (3b) (1"R/S) = 3:2

2-Methyl-2-[(2,2,3-trimethylcyclopent-3-enyl)methoxy]propanal **(4a)** from Example 2 (1.0 g, 4.8 mmol) dissolved in THF (2 ml) was added dropwise during 30 min. to a 2.9 M THF solution of methylmagnesium chloride (2.0 ml, 5.8 mmol) at 0°C. The reaction mixture was poured into aqueous saturated citric acid solution-ice and the aqueous layer extracted with MTBE (3 x). The combined organic phases were washed with water to pH 7, dried (MgSO₄), concentrated in vacuo and purified by flash chromatography (MTBE/hexane 1:9) to afford 3-methyl-3-[(2,2,3-trimethylcyclopent-3-enyl)methoxy]butan-2-ol (3b, 0.7 g, 65% yield, colourless oil) as a ~ 1:1 mixture of diastereomeric pairs of enantiomers.
¹H NMR: *δ* 5.20 (m, 2H), 3.65 *(dq, J =* 6.5, 3.0, 1H), 3.63 *(dq, J =* 6.5, 3.0, 1 H), 3.50 (dd, *J* = 8.6, 6.9, 1 H), 3.48 (dd, *J* = 8.6, 7.1, 1H), 3.38 (t, *J* = 7.2, 1H), 3.35 (t, *J* = 7.1, 1H), 2.66 (d, *J* = 3.0, 1 H), 2.64 (d, J = 3.0, 1 H), 2.33-2.24 (m, 2H), 2.11-2.02 (m, 2H), 1.95-1.85 (m, 2H), 1.59 (dt, *J* = 2.5, 1.6, 6H), 1.13 (s, 6H), 1.12 (d, *J* = 6.5, 6H), 1.11 (s, 3H), 1.09 (s, 3H), 1.07 (s, 3H), 1.06 (s, 3H), 0.85 (2s, 6H). ¹³C NMR: *δ* 148.2 (2s), 121.3 (d), 121.2 (d), 77.2 (2s), 73.5 (d), 72.9 (d), 62.3 (t), 62.2 (t), 49.8 (d), 49.7 (d), 46.4 (2s), 33.6 (2t), 27.0 (q), 26.9 (q), 21.3 (q), 21.2 (q), 19.8 (2q), 18.6 (q), 18.1 (q), 16.8 (q), 16.7 (q), 12.2 (2q). MS: 208(1), 181(7), 139(3), 123(100), 122(28), 107(48), 95(14), 87(13), 81(45), 69(12), 57(13), 45(10), 43(14), 41(16).
Odour description: ambery, green, dry, sandalwood, powdery.

### Example 4: 3-[1-(2,2,3-Trimethylcyclopent-3-enyl)ethoxy]butan-2-ol (3c); (1"R/S) = 3:2

### a) 1-(2,2,3-Trimethylcyclopent-3-enyl)ethanol (2b); (1"R/S) = 3:2

**2b** was obtained according to Example 3, starting from campholytic aldehyde 1 of the corresponding enantiomer ratio. The crude product was purified by distillation under reduced pressure; 19% yield, colourless oil, 1:1.4 mixture of diastereomeric pairs of enantiomers. Analytical samples of both pairs were obtained by flash chromatography (MTBE/hexane 1:4) separation.
Major diastereoisomer: ¹H NMR: *δ* 5.17 (m, 1 H), 3.90 (dqb, J = 8.3, 6.2, 1 H), 2.18-2.06 (m, 1H), 1.85-1.75 (m, 2H), 1.58 (dt, J = 2.5, 1.5, 3H), 1.45 (sb, 1 H), 1.19 (d, J = 6.2, 3H), 1.17 (s, 3H), 0.97 (s, 3H). ¹³C NMR: *δ* 149.0 (s), 120.6 (d), 69.8 (d), 57.5 (d), 47.0 (s), 33.2 (t), 27.6 (q), 23.8 (q), 19.6 (q), 12.3 (q). MS: 154(M⁺, 4), 139(1), 136(15), 121(40), 109(5), 105(4), 96(8), 95(100), 93(8), 91(6), 79(9), 67(17), 55(8), 45(11), 43(9), 41(8).
Minor diastereoisomer: ¹H NMR: *δ* 5.26 (m, 1H), 3.93 (qi, *J* = 6.3, 1H), 2.38-2.29 (m, 1 H), 2.25-2.16 (m, 1 H), 1.80 (dt, J = 8.3, 6.3, 1 H), 1.64 (sb, 1 H), 1.59 (dt, J = 2.5, 1.6, 3H), 1.25 (d, J = 6.3, 3H), 1.04 (s, 3H), 0.92 (s, 3H). ¹³C NMR: *δ* 148.1 (s), 121.6 (d), 68.6 (d), 56.7 (d), 46.5 (s), 31.5 (t), 26.9 (q), 23.4 (q), 20.0 (q), 12.3 (q). MS: 154(M⁺, 4), 139(1), 136(21), 122(10), 121(100), 107(6), 109(5), 105(11), 95(73), 93(14), 91 (10), 79(13), 67(19), 55(10), 45(12), 43(11), 41(11).

### b) 3-[1-(2,2,3-Trimethylcyclopent-3-enyl)ethoxy]butan-2-ol (3c); (1"R/S) = 3:2

**3c** was obtained by reacting 1-(2,2,3-trimethylcyclopent-3-enyl)ethanol **(2b)** with *cis*/*trans*-2,3-epoxybutane according to the experimental procedure of Example 1; 19% yield, colourless oil; mixture of diastereomeric pairs of enantiomers (4 pairs of approximately 1:1:1:1 ratio constituted ∼90% of the mixture). Flash chromatography (MTBE/hexane 1:4) separation afforded 2 mixtures of 2 pairs each.
¹H NMR: 2 first eluted (FC) enantiomer pairs: *δ* 5.28 (m, 1H), 5.17 (m, 1H), 3.74 (dq, J = 9.3, 5.9, 1H), 3.58 (dq, J = 7.9, 6.0, 1H), 3.56-3.44 (m, 2H), 3.38 (dq, J = 7.0, 6.2, 1 H), 3.26 (dq, *J* = 7.2, 6.0, 1 H), 2.88 (d, *J* = 1.9, 1 H), 2.81 (d, *J* = 2.2, 1 H), 2.34 (m, 1 H), 2.19-2.03 (m, 2H), 1.95-1.78 (m, 3H), 1.58 (m, 6H), 1.21 (d, J= 6.0, 3H), 1.15 (d, *J =* 6.0, 3H), 1.14 (d, *J =* 6.0, 3H), 1.12 (s, 3H), 1.11 (d, *J =* 6.2, 3H), 1.10 (d, *J =* 5.9, 3H), 1.07 (d, *J =* 5.9, 3H), 1.06 (s, 3H), 0.90 (s, 3H), 0.86 (s, 3H).
¹³C NMR: 2 first eluted (FC) enantiomer pairs: *δ* 149.3 (s), 147.8 (s), 121.6 (d), 120.5 (d), 75.9 (d), 74.6 (d), 72.6 (d), 72.1 (d), 71.2 (d), 71.0 (d), 56.2 (d), 56.0 (d), 46.8 (s), 46.0 (s), 33.4 (t), 33.3 (t), 27.3 (q), 26.8 (q), 19.7 (q), 19.4 (q), 19.2 (q), 18.5 (q), 18.4 (q), 18.1 (q), 15.4 (q), 15.0 (q), 12.3 (2q); 2 second eluted (FC) enantiomer pairs: *δ* 148.4 (s), 147.0 (s), 121.5 (d), 121.0 (d), 79.6 (d), 77.7 (d), 77.4 (d), 75.5 (d), 71.3 (d), 70.8 (d), 56.7 (d), 55.1 (d), 46.4 (s), 46.1 (s), 33.9 (t), 31.9 (t), 27.2 (q), 26.9 (q), 21.9 (q), 20.0 (q), 19.5 (q), 18.5 (q), 18.4 (q), 18.2 (q), 17.6 (q), 16.9 (q), 12.3 (2q). MS: 2 first eluted (GC) enantiomer pairs: 181(2), 137(65), 136(46), 121(100), 107(15), 95(34), 81(14), 73(54), 57(16), 55(25), 45(10), 43(16), 41(13); third eluted (GC) enantiomer pair: 181(1), 137(100), 136(52), 121(61), 107(16), 95(53), 91(14), 81(21), 73(93), 57(22), 55(34), 45(22), 43(20), 41(17); fourth eluted (GC) enantiomer pair: 181(1), 137(40), 136(36), 121(100), 107(11), 95(24), 81 (10), 73(27), 57(12), 55(17), 45(12), 43(12), 41(10).
Odour description: woody, sandalwood, patchouli.

The following compounds (Examples 5 - 9) have been prepared according to the general procedure given in Example 1, starting from *α*-campholytic alcohol (**2a**, (R/S) = 3:2 or 1:9) or 1-(2,2,3-trimethylcyclopent-3-enyl)ethanol **(2b)** and an appropriate oxirane.

### Example 5: 3-[(2,2,3-Trimethylcyclopent-3-enyl)methoxy]butan-2-ol (3d); (1"R/S) = 3:2; mixture of 4 diastereomeric pairs of enantiomers (approximately - 3:3:1:1)

2 main pairs of enantiomers: ¹H NMR: *δ* 5.23-5.19 (m, 2H), 3.77 (dd, J = 8.9, 6.6, 1 H), 3.59 (dd, *J* = 9.1, 7.7, 1H), 3.58-3.50 (m, 2H), 3.43 (dd, J = 9.1, 6.6, 1H), 3.27 (dd, *J* = 8.9, 7.7, 1H), 3.13 (dq, *J* = 15.3, 6.2, 1H), 3.13 (qb, *J =* 6.2, 1,1, 1H), 2.86 (d, J = 2.3, 1 H), 2.81 (d, J = 2.5, 1 H), 2.38-2.27 (m, 2H), 2.18-2.09 (m, 2H), 1.99-1.88 (m, 2H), 1.59 (dt, J = 2.5, 1.6, 6H), 1.15 (d, *J =* 6.3, 3H), 1.14 (d, *J =* 6.3, 3H), 1.12 (d, *J =* 6.2, 3H), 1.115 (d, *J=* 6.2, 3H), 1.07 (s, 3H), 1.0.6 (s, 3H), 0.85 (s, 3H), 0.84 (s, 3H). ¹³C NMR: *δ* 148.0 (s), 147.9 (s), 121.3 (2d), 80.9 (d), 80.3 (d), 71.1 (d), 71.0 (d), 70.5 (t), 70.1 (t), 49.6 (d), 49.4 (d), 46.4 (s), 46.3 (s), 33.7 (2t), 26.9 (q), 26.8 (q), 19.9 (q), 19.7 (q), 18.5 (q), 18.4 (q), 15.2 (q), 15.1 (q), 12.2 (2q). MS: 167(4), 123(46), 122(38), 107(100), 95(12), 91(13), 81(32), 79(10), 73(11), 67(9), 57(10), 55(16), 45(9), 43(10), 41(11).
2 minor pairs of enantiomers: ¹³C NMR: *δ* 148.1 (s), 148.0 (s), 121.2 (2d), 79.0 (d), 78.8 (d), 70.3 (d), 70.1 (d), 69.3 (t), 69.1 (t), 49.5 (d), 49.4 (d), 46.4 (s), 46.3 (s), 33.5 (2t), 26.9 (q), 26.8 (q), 19.9 (q), 19.7 (q), 17.7 (q), 17.6 (q), 13.7 (q), 13.4 (q), 12.2 (2q). MS: 167(4), 123(51), 122(41), 107(100), 95(13), 91(14), 81(36), 79(11), 73(12), 67(10), 57(12), 55(17), 45(10), 43(12), 41(13).
Odour description: woody, sandalwood, lactonic, coumarin, floral, more woody/sandalwood than **3d'** from Example 6.

### Example 6: 3-[(2,2,3-Trimethylcyclopent-3-enyl)methoxy]butan-2-ol (3d'); (1"R/S) = 1:9; mixture of 2 diastereomeric pairs of enantiomers (approximately ~ 1:1) corresponding to the main pairs of enantiomers from Example 5 (same spectra), contains 7% of the other pair

Odour description: woody, sandalwood, lactonic, coumarin, floral, more floral than **3d** from Example 5.

### Example 7: 2:1 Mixture of 1-[(2,2,3-trimethylcyclopent-3-enyl)methoxylbutan-2-ol and 2-[(2,2,3-trimethylcyclopent-3-enyl)methoxy]butan-1-ol (3e'); (1"R/S) = 1:9; diastereomer ratio ∼1:1

Major regioisomer (2 pairs of enantiomers): ¹³C NMR: *δ* 148.1 (s), 148.0 (s), 121.3 (d), 121.2 (d), 75.0 (t), 74.9 (t), 72.8 (2t), 71.7 (d), 71.6 (d), 49.1 (2d), 46.4 (2s), 33.5 (t), 33.4 (t), 26.8 (2q), 26.1 (2t), 19.8 (2q), 12.2 (2q), 9.9 (q), 9.8 (q). MS: 123(8), 122(25), 108(9), 107(100), 93(6), 91(9), 81(9), 79(6), 73(3), 67(5), 55(12), 41(7).
Minor regioisomer (2 pairs of enantiomers): ¹³C NMR: δ 148.1 (s), 148.0 (s), 121.3 (d), 121.2 (d), 81.5 (d), 81.3 (d), 70.9 (t), 70.8 (t), 63.8 (t), 63.6 (t), 49.6 (d), 49.5 (d), 46.4 (s), 46.3 (s), 33.9 (t), 33.6 (t), 26.8 (q), 26.7 (q), 23.5 (t), 23.3 (t), 19.9 (q), 19.7 (q), 12.2 (2q), 9.7 (q), 9.6 (q). MS: 181(3), 123(28), 122(32), 107(100), 95(10), 91(11), 81(22), 57(8), 55(18), 43(9), 41 (10).
Odour description: woody, sandalwood, creamy, green, honey.

### Example 8: 2-Methyl-2-[1-(2,2,3-trimethylcyclopent-3-enyl)ethoxy]propan-1-ol (3f); (1"R/S) = 3:2; diastereomer ratio ∼1:1.1

Major diastereoisomer (first eluted): ¹³C NMR: *δ* 148.7 (s), 120.9 (d), 75.1 (s), 71.5 (t), 69.8 (d), 56.3 (d), 46.6 (s), 32.5 (t), 27.3 (q), 23.6 (q), 23.3 (q), 21.7 (q), 19.8 (q), 12.4 (q). MS: 195(1), 138(10), 137(100), 136(46), 121(66), 109(28), 107(12), 95(80), 93(15), 91(16), 81(21), 73(97), 67(17), 59 (6), 57(21), 55(34), 43(24), 41(26).

Minor diastereoisomer (second eluted): ¹³C NMR: *δ* 147.9 (s), 121.6 (d), 75.3 (s), 71.3 (t), 69.8 (d), 57.0 (d), 45.9 (s), 34.7 (t), 26.9 (q), 23.8 (q), 23.5 (q), 22.7 (q), 19.3 (q), 12.4 (q). MS: 195(1), 153 (2), 137(49), 136(36), 121(100), 109(34), 107(10), 95(42), 93(13), 91(12), 81(13), 73(35), 67(13), 59 (4), 57(14), 55(22), 43(19), 41(15).
Odour description: woody, sandalwood, cedarwood, slightly agrestic and patchouli.

### Example 9: 4:1 Mixture of 1-[1-(2,2,3-trimethylcyclopent-3-enyl)ethoxy]propan-2-ol and 2-[1-(2,2,3-trimethylcyclopent-3-enyl)ethoxy]propan-1-ol (3g); (1"R/S) = 3:2)

Major regioisomer (4 pairs of enantiomers): ¹³C NMR: *δ* 149.0 (s), 148.9 (s), 147.6 (2s), 121.6 (d), 121.5 (d), 120.5 (2d), 77.7 (d), 77.5 (d), 77.4 (d), 76.7 (d), 76.1 (d), 73.9 (d), 73.5 2(d), 73.4 (d), 66.9 (t), 66.8 (t), 66.7 (t), 66.6 (t), 56.1 (d), 56.0 (d), 55.6 (d), 55.5 (d), 47.0 (s), 46.9 (s), 46.3 (2s), 33.2 (t), 31.1 (t), 32.1 (2t), 27.6 (q), 27.5 (q), 26.9 (2q), 20.1 (q), 20.0 (q), 19.8 (q), 19.7 (q), 19.2 (2q), 19.0 (q), 18.8 (q), 18.7 (q), 18.5 (q), 18.2 (q), 18.1 (q), 12.3 (4q). MS: 137(11), 136(37), 122(10), 121(100), 107(11), 103(12), 95(13), 93(9), 91(9), 79(8), 67(6), 59(68), 55(7), 45(12), 43(9), 41(12).
Minor regioisomer (4 pairs of enantiomers): ¹³C NMR (selected peaks): 67.1 (t), 66.8 (t), 66.3 (2t). MS: 181(1), 137(30), 136(40), 122(10), 121(100), 107(11), 103(12), 95(24), 93(12), 91(12), 81(9), 79(10), 77(8), 67(9), 59(64), 57(10), 55(10), 45(15), 43(12), 41(16).
Odour description: woody, patchouli, sandalwood, agrestic, creamy.

### Example 10: 2-Methyl-2-[(1,2,2-trimethylbicyclo[3.1.0]hex-3-yl)methoxy]propan-1-ol (5a); (3"R/S) = 3:2

15% Triethylaluminium solution in hexane (11.5 ml, 10.3 mmol) was added during 1 h to a solution of 2-methyl-2-[(2,2,3-trimethylcyclopent-3-enyl)methoxy]propan-1-ol (**3a**) from Example 1 (1.0 g, 4.7 mmol) and diiodomethane (2.9 g, 11 mmol) in hexane (40 ml) at 10°C. After additional 90 h stirring at room temperature, the reaction mixture was poured into ice-water, acidified with 1 N sulphuric acid to pH 5 and the aqueous layer extracted with MTBE (3 x). The combined organic phases were washed with water, dried (MgSO₄) and concentrated in vacuo. The separation of the unreacted **3a** by chromatography proved difficult. Sodium acetate (0.2 g, 2.4 mmol) and MCPBA (0.6 g, 70% pure, 2.4 mmol) were added to the crude product (0.9 g) dissolved in dichloromethane (2 ml) and, after additional 1 h stirring at room temperature, the reaction mixture was poured into ice-water, basified with saturated aqueous sodium bicarbonate solution and the aqueous layer extracted with hexane (3 x). The combined organic phases were washed with water, dried (MgSO₄), concentrated in vacuo and purified by flash chromatography (MTBE/hexane 1:5) to afford 2-methyl-2-[(1,2,2-trimethylbicyclo[3.1.0]hex-3-yl)methoxy]propan-1-ol; (**5a**, 0.3 g, 28% yield, colourless oil) as one pair (GC purity 96.5%) of enantiomers.
¹H NMR: *δ* 3.40 (d, *J_{AB}* = 10.8, 1H), 3.36 (d, *J_{AB} =* 10.8, 1 H), 3.32 (dd, *J =* 8.7, 5.7, 1H), 3.17 (dd, J = 8.7, 7.0, 1H), 2.07 (sb, 1 H), 1.75 (m, 1 H), 1.49-1.38 (m, 2H), 1.14 (s, 3H), 1.13 (s, 3H), 1.02 (s, 3H), 0.99 (m, 1H), 0.98 (s, 3H), 0.81 (s, 3H), 0.45 (m, 1H), 0.04 (dd, *J =* 7.8, 4.8, 1 H). ¹³C NMR: *δ* 74.5 (s), 69.8 (t), 62.8 (t), 44.4 (d), 40.9 (s), 31.7 (s), 30.8 (t), 23.8 (q), 22.6 (d), 21.9 (q), 21.5 (q), 19.6 (q), 16.9 (q), 14.0 (t). MS: 211(1), 195(11), 154(9), 139(60), 137(76), 136(37), 123(23), 121(90), 109(25), 107(29), 95(95), 93(50), 81(100), 73(76), 69(52), 67(40), 57(52), 55(82), 43(40), 41(56);
Odour description: sandalwood, woody, powdery, green, lactonic.

### Example 11: 2-Methyl-2-[(2,2,3-trimethylcyclopentyl)methoxy]propan-1-ol (6a); (1"R/S) = 3:2

A solution of 2-methyl-2-[(2,2,3-trimethylcyclopent-3-enyl)methoxy]propan-1-ol **(3a)** from Example 1 (1.0 g, 4.7 mmol) in ethanol (10 ml) was hydrogenated during 3 hours in the presence of palladium on activated carbon (5%, 0.55 g), at room temperature and under normal pressure. The catalyst was filtered off, the solvent evaporated in vacuo and the residue was distilled (bulb-to-bulb) to afford 2-methyl-2-[(2,2,3-trimethylcyclopentyl)methoxy]propan-1-ol (3b, 0.65 g, 64% yield, colourless liquid) as a ~ 9:1 mixture of diastereomeric pairs of enantiomers.
Main pair of enantiomers: ¹H NMR: *δ* 3.42 (dd, J = 8.5, 5.9, 1H), 3.42-3.37 (m, 2H), 3.19 (dd, J = 8.5, 7.8, 1 H), 2.22 (tb, J = 5.7, 1 H), 1.87-1.66 (m, 3H), 1.61-1.49 (m, 1 H), 1.28-1.17 (m, 2H), 1.16 (s, 3H), 1.15 (s, 3H), 0.96 (s, 3H), 0.82 (d, *J =* 6.8, 3H), 0.56 (s, 3H). ¹³C NMR: *δ* 74.5 (s), 69.7 (t), 63.2 (t), 50.4 (d), 45.6 (d), 41.7 (s), 30.1 (t), 26.6 (q), 26.5 (t), 21.9 (q), 21.5 (q), 14.6 (q), 13.3 (q). MS: 183(7), 126(7), 125(77), 109(6), 83(34), 73(12), 70(8), 69(100), 67(9), 59(11), 57(22), 55(35), 43(15), 41(25).
Minor pair of enantiomers: ¹³C NMR: *δ* 74.5 (s), 69.8 (t), 63.4 (t), 48.6 (d), 44.3 (d), 41.5 (s), 31.6 (t), 27.4 (t), 24.4 (q), 23.6 (q), 21.8 (q), 21.6 (q), 15.4 (q). MS: 183(7), 126(8), 125(75), 109(12), 83(36), 73(14), 70(9), 69(100), 67(8), 59(10), 57(20), 55(32), 43(13), 41(26).
Odour description: woody, agrestic, floral, fruity, weaker than the unsaturated analogue **3a**.

### Example 12: Floral-woody fragrance composition for eau de Cologne

| Ingredient | parts by weight |
|---|---|
| Adoxal (2,6,10-trimethyl-9-undecenal) | 2 |
| Ambrettolide (oxacycloheptadec-10-en-2-one) | 14 |
| Aurantiol^{®} (methyl N-3,7-dimethyl-7-hydroxyoctylidenanthranilate) | 15 |
| Bourgeonal (4-(1,1-dimethylethyl)benzenepropanal) | 3 |
| Cashmeran^{®} (1,1,2,3,3-pentamethyl-1,2,3,5,6,7-hexahydro-(4H)-inden-4-one) | 8 |
| Cepionate^{®} (methyl 2-(3-oxo-2-pentylcyclopentyl)acetate) | 150 |
| Cosmone™ (3-methyl-5-cyclotetradecen-1-one) | 10 |
| Damascenone 10% in DEP (diethyl phthalate) | 3 |
| (1-(2,6,6-trimethyl-1,3-cyclohexadien-1-yl)-2-buten-1-one) | |
| delta-Decalactone | 8 |
| Ethylene brassylate | 45 |
| Eugenol | 7 |
| Florol^{®} (2-isobutyl-4-methyltetrahydro-2H-pyran-4-ol) | 100 |
| Galaxolide^{®} 50% in DEP | 110 |
| (4,6,6,7,8,8-hexamethyl-1,3,4,6,7,8-hexahydrocyclopenta(g)-2-benzopyran) | |
| Gardenol (α-methylbenzyl acetate) | 5 |
| Georgywood | 35 |
| (1-(1,2,3,4,5,6,7,8-octahydro-1,2,8,8-tetramethyl-2-naphthalenyl)ethanone) | |
| cis-3-Hexenyl acetate | 1 |
| cis-3-Hexenyl salicylate | 45 |
| Indolene | 10 |
| Linalool | 55 |
| Methyl isoeugenol | 15 |
| Okoumal^{®} 10% in DEP | 4 |
| (2,4-dimethyl-2-(1,1,4,4-tetramethyltetralin-6-yl)-1,3-dioxolane) | |
| Compound **3d'** from Example 6 | 55 |
| | 700 |

3-[(2,2,3-Trimethylcyclopent-3-enyl)methoxy]butan-2-ol (3d') from Example 6 brings to the accord a creamy note and enhances the sensual tuberose-like one. It augments the composition's volume, roundness and radiance.

## Claims

1. A compound of formula (I) 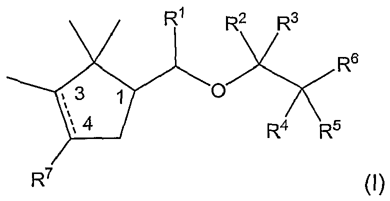 wherein:
R¹, R², R³, and R⁴ are independently selected from hydrogen, methyl or ethyl;
R⁵ is hydrogen, methyl or ethyl; and R⁶ is hydroxyl; or
R⁵ and R⁶ form together with the carbon atom to which they are attached a carbonyl group;
R⁷ is hydrogen and the bond between C-3 and C-4 is a single bond or the dotted line together with the bond between C-3 and C-4 represents a double bond; or
R⁷ is methylene forming with C-3 and C-4 a cyclopropane ring.

2. A compound according to claim 1 enriched in a compound of formula (A)

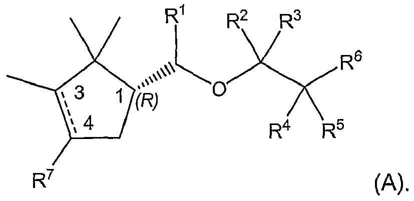

3. A compound according to claim 1 enriched in a compound of formula (B)

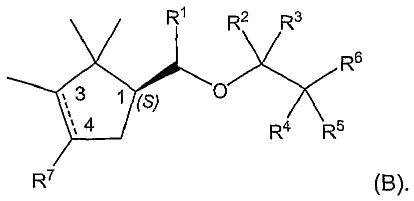

4. A compound according to claim 1 selected from the group consisting of
2-methyl-2-[(2,2,3-trimethylcyclopent-3-enyl)methoxy]propan-1-ol,
2-methyl-2-[(2,2,3-trimethylcyclopent-3-enyl)methoxy]propanal,
3-methyl-3-[(2,2,3-trimethy(cyclopent-3-enyl)methoxy]butan-2-ol,
3-[1-(2,2,3-trimethylcyclopent-3-enyl)ethoxy]butan-2-ol,
3-[(2,2,3-trimethylcyclopent-3-enyl)methoxy]butan-2-ol,
1-[(2,2,3-trimethylcyclopent-3-enyl)methoxy]butan-2-ol,
2-[(2,2,3-trimethylcyclopent-3-enyl)methoxy]butan-1-ol,
2-methyl-2-[1-(2,2,3-trimethylcyclopent-3-enyl)ethoxy]propan-1-ol,
1-[1-(2,2,3-trimethylcyclopent-3-enyl)ethoxy]propan-2-ol,
2-[1-(2,2,3-trimethylcyclopent-3-enyl)ethoxy]propan-1-ol,
2-methyl-2-[(1,2,2-trimethylbicyclo[3.1.0]hex-3-yl)methoxy]propan-1-ol, and
2-methyl-2-[(2,2,3-trimethylcyclopentyl)methoxy]propan-1-ol.

5. A fragrance composition comprising a compound as defined in any one of the preceding claims.

6. A fragrance application comprising
a) a compound of formula (I) as defined in any one of the claims 1 to 4; and
b) a consumer product base.

7. A fragrance application according to claim 6 wherein the consumer product base is selected from the group consisting of fine fragrance, household product, laundry product, body care product and cosmetic.

8. The use as fragrance ingredient of a compound as defined in any one of the claims 1 to 4.

9. A method of manufacturing a fragrance composition, comprising the incorporation of an effective amount of a compound of formula (I) as defined in any one of the claims 1 to 4, or a mixture thereof to a base material.

10. A method of improving, enhancing or modifying a fragrance of a fragrance composition or fragrance application comprising the step of incorporating an effective amount of a compound of formula (I) as defined in any one of the claims 1 to 4, or a mixture thereof to a base material.

## Patentansprüche

1. Verbindung der Formel (I), 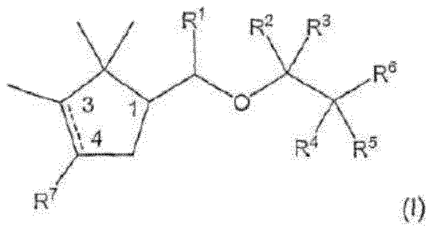 in der
R¹, R², R³ und R⁴ unabhängig aus Wasserstoff, Methyl oder Ethyl ausgewählt sind;
R⁵ Wasserstoff, Methyl oder Ethyl bedeutet und R⁶ Hydroxyl bedeutet; oder
R⁵ und R⁶ gemeinsam mit dem Kohlenstoffatom, an das sie gebunden sind, eine Carbonylgruppe bilden,
R⁷ Wasserstoff bedeutet und die Bindung zwischen C-3 und C-4 eine Einfachbindung ist oder die gestrichelte Linie gemeinsam mit der Bindung zwischen C-3 und C-4 eine Doppelbindung bedeutet; oder
R⁷ Methylen bedeutet, das mit C-3 und C-4 einen Cyclopropanring bildet.

2. Verbindung nach Anspruch 1, die an einer Verbindung der Formel (A) 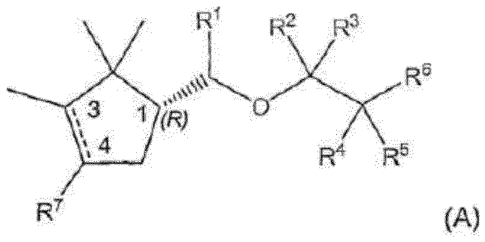 angereichert ist.

3. Verbindung nach Anspruch 1 die an einer Verbindung der Formel (B) 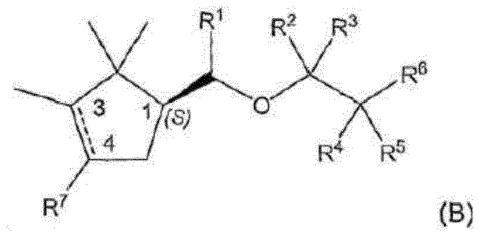 angereichert ist.

4. Verbindung nach Anspruch 1, ausgewählt aus der Gruppe bestehend aus
2-Methyl-2-[(2,2,3-trimethylcyclopent-3-enyl)-methoxy]propan-1-ol,
2-Methyl-2-[(2,2,3-trimethylcyclopent-3-enyl)-methoxy]propanal,
3-Methyl-3-[(2,2,3-trimethylcyclopent-3-enyl)-methoxy]butan-2-ol,
3-[1-(2,2,3-trimethylcyclopent-3-enyl)ethoxy]-butan-2-ol,
3-[(2,2,3-Trimethylcyclopent-3-enyl)methoxy]butan-2-ol,
1-[(2,2,3-Trimethylcyclopent-3-enyl)methoxy]butan-2-ol,
2-[(2,2,3-Trimethylcyclopent-3-enyl)methoxy]butan-1-ol,
2-Methyl-2-[1-(2,2,3-trimethylcyclopent-3-enyl)-ethoxy]propan-1-ol,
1-[1-(2,2,3-Trimethylcyclopent-3-enyl)ethoxy]-propan-2-ol,
2-[1-(2,2,3-Trimethylcyclopent-3-enyl)ethoxy]-propan-1-ol,
2-Methyl-2-[(1,2,2-trimethylbicyclo[3.1.0]hex-3-yl)methoxy]propan-1-ol, und
2-Methyl-2-[(2,2,3-trimethylcyclopentyl)methoxy]-propan-1-ol.

5. Duftstoffzusammensetzung, die eine wie in einem der vorhergehenden Ansprüche definierte Verbindung umfasst.

6. Duftstoffanwendung, die
a) eine wie in einem der Ansprüche 1 bis 4 definierte Verbindung der Formel (I) und
b) eine Konsumentenproduktgrundlage umfasst.

7. Duftstoffanwendung nach Anspruch 6, wobei die Konsumentenproduktgrundlage aus der Gruppe bestehend aus Parfum, Haushaltsprodukt, Waschmittel, Körperpflegeprodukt und Kosmetikum ausgewählt ist.

8. Verwendung einer wie in einem der Ansprüche 1 bis 4 definierten Verbindung als Duftstoffbestandteil.

9. Verfahren zur Herstellung einer Duftstoffzusammensetzung, bei dem eine wirksame Menge einer wie in einem der Ansprüche 1 bis 4 definierten Verbindung der Formel (I) oder eine Mischung davon in ein Basismaterial eingearbeitet wird.

10. Verfahren zum Verbessern, Fördern oder Modifizieren eines Dufts einer Duftstoffzusammensetzung oder Duftstoffanwendung, bei dem man eine wirksame Menge einer wie in einem der Ansprüche 1 bis 4 definierten Verbindung der Formel (I) oder eine Mischung davon in ein Basismaterial einarbeitet.

## Revendications

1. Composé de formule (I) 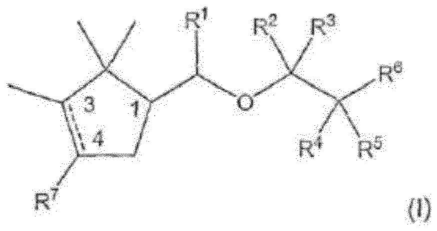 dans lequel :
R¹, R², R³ et R⁴ sont indépendamment choisis parmi hydrogène, méthyle ou éthyle ;
R⁵ est hydrogène, méthyle ou éthyle ; et R⁶ est hydroxyle ; ou
R⁵ et R⁶ forment conjointement avec l'atome de carbone auquel ils sont liés un groupe carbonyle ;
R⁷ est hydrogène et la liaison entre C-3 et C-4 est une simple liaison ou le trait pointillé conjointement avec la liaison entre C-3 et C-4 représente une double liaison ; ou
R⁷ est méthylène formant avec C-3 et C-4 un cycle cyclopropane.

2. Composés selon la revendication 1 enrichi en un composé de formule (A)

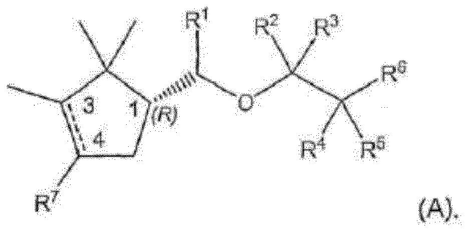

3. Composé selon la revendication 1 enrichi en un composé de formule (B)

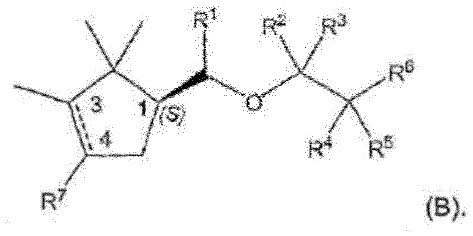

4. Composé selon la revendication 1 choisi dans le groupe constitué de
2-méthyl-2-[(2,2,3-triméthylcyclopent-3-ényl)méthoxylpropan-1-ol,
2-méthyl-2-[(2,2,3-triméthylcyclopent-3-ényl)méthoxy]propanal,
3-methyl-3-[(2,2,3-triméthylcyclopent-3-ényl)méthoxy]butan-2-ol,
3-[1-(2,2,3-triméthylcyclopent-3-ényl)éthoxy]butan-2-ol,
3-[(2,2,3-triméthylcyclopent-3-ényl)méthoxy]butan-2-ol,
1-[(2,2,3-triméthylcyclopent-3-ényl)méthoxy]butan-2-ol,
2-[(2,2,3-triméthylcyclopent-3-ényl)méthoxy]butan-1-ol,
2-méthyl-2-[1-(2,2,3-triméthylcyclopent-3-ényl)éthoxy]propan-1-ol,
1-[1-(2,2,3-triméthylcyclopent-3-ényl)éthoxylpropan-2-ol,
2-[1-(2,2,3-triméthylcyclopent-3-ényl)éthoxylpropan-1-ol,
2-méthyl-2-[(1,2,2-triméthylbicyclo[3.1.0]hex-3-yl)méthoxylpropan-1-ol, et
2-méthyl-2-[(2,2,3-triméthylcyclopentyl)méthoxy]propan-1-ol.

5. Composition de parfum comprenant un composé tel que défini dans l'une quelconque des revendications précédentes.

6. Application de parfum comprenant
a) un composé de formule (I) tel que défini dans l'une quelconque des revendications 1 à 4 ; et
b) une base de produit de consommation.

7. Application de parfum selon la revendication 6 dans laquelle la base de produit de consommation est choisie dans le groupe constitué d'un parfum fin, un produit domestique, un produit de blanchisserie, un produit de soin du corps et un cosmétique.

8. Utilisation en tant que composant de parfum d'un composé tel que défini dans l'une quelconque des revendications 1 à 4.

9. Procédé de fabrication d'une composition de parfum, comprenant l'incorporation d'une quantité efficace d'un composé de formule (I) tel que défini dans l'une quelconque des revendications 1 à 4, ou un mélange de celui-ci avec un matériau de base.

10. Procédé d'amélioration, de renforcement de modification d'un parfum d'une composition de parfum ou application de parfum comprenant l'étape d'incorporation d'une quantité efficace d'un composé de formule (I) tel que défini dans l'une quelconque des revendications 1 à 4, ou un mélange de celui-ci avec un matériau de base.
